# EUROPEAN PATENT SPECIFICATION

(11) **EP 1 196 154 B1**
(45) Date of publication and mention of the grant of the patent: **13.09.2006**
(21) Application number: 00949378.4
(22) Date of filing: 17.07.2000
(51) Int. Cl.: A61K 31/00

(54) **INHIBITION OF RENAL UPTAKE OF RADIOMOLECULES WITH A COMBINATION OF LYSINE AND ARGININE**
HEMMUNG DER AUFNAHME SCHÄDIGENDER PROTEINE DURCH DIE NIEREN MIT EINER KOMBINATION VON LYSIN UND ARGININ
INHIBITION DE L'ABSORPTION RENALE DE MOLECULES POUVANT POTENTIELLEMENT ENDOMMAGER LES REINS A L'AIDE D'UNE COMBINAISON DE LYSINE ET D'ARGININE

(30) Priority: 16.07.1999 US 144106 P
(43) Date of publication of application: 17.04.2002
(73) Proprietor: MALLINCKRODT, INC., St. Louis, MO 63134 (US)
(72) Inventor: KRENNING, Eric, Paul, NL-3062 JC Rotterdam (NL); DE JONG, Marion, NL-3135 PA Vlaardingen (NL); VALKEMA, Roelf, NL-2341 CK Oegstgeest (NL)
(74) Representative: Hooiveld, Arjen Jan Winfried
(86) International application number: PCT/EP2000/006917
(87) International publication number: WO 2001/005383

(56) References cited:
- EP-A- 0 747 395
- WO-A-91/04755
- WO-A-96/29087
- WO-A-97/46099
- HAMMOND P J ET AL: "Amino acid infusion blocks renal tubular uptake of an indium-labelled somatostatin analogue" BRITISH JOURNAL OF CANCER,GB,LONDON, vol. 67, June 1993 (1993-06), pages 1437-1439, XP002105271 ISSN: 0007-0920
- BEHR T M ET AL: "REDUCTION OF THE RENAL UPTAKE OF RADIOLABELED MONOCLONAL ANTIBODY FRAGMENTS BY CATIONIC AMINO ACIDS AND THEIR DERIVATIVES" CANCER RESEARCH,AMERICAN ASSOCIATION FOR CANCER RESEARCH, BALTIMORE, MD,US, vol. 55, no. 17, 1 September 1995 (1995-09-01), pages 3825-3834, XP000946492 ISSN: 0008-5472
- PIMM M V ET AL: "Prevention of renal tubule re-absorption of radiometal (indium-111) labelled Fab fragment of a monoclonal antibody in mice by systemic administration of lysine" EUROPEAN JOURNAL OF NUCLEAR MEDICINE,DE,BERLIN, vol. 21, no. 7, July 1994 (1994-07), pages 663-665, XP002105272 ISSN: 0340-6997
- BEHR T. M. ET AL: "Reduction of renal uptake of monoclonal antibody fragments by aminoacid infusion" J. NUCL. MED., vol. 37, no. 5, May 1996 (1996-05), pages 829-833, XP000978766
- KRENNING E. P. ET AL: "Radiolabelled somatostatin analogue(s) for peptide receptor scintigraphy and radionuclide therapy" ANNALS OF ONCOLOGY, vol. 10, no. Suppl 2, 1999, pages s23-s29, XP000978221
- BEHR T. M.; GOLDENBERG D. M.: "Improved prospects for cancer therapy with radiolabeled antibody fragments and peptides?" J. NUCL. MED., vol. 37, no. 5, May 1996 (1996-05), pages 834-836, XP000978767

## Description

The present invention relates to the inhibition of renal uptake of molecules that are potentially damaging for the kidney.

Radionuclide labeled peptides and also monoclonal antibodies or their fragments and other compounds like certain antibiotics undergo undesired renal uptake and cellular retention leading to a high kidney dose, or concentration. In the case of diagnosis or therapy with radiolabeled proteinaceous compounds, like peptides and monoclonal fragments, the uptake of these compounds by the kidneys leads to a reduction in the detection sensitivity of perirenal tumors or an increase in kidney radiotoxicity.

In certain other situations a general overload of proteins in the kidney can occur, such as in the case of amino acid metabolism diseases, induced catabolism or prolonged physical exercise, e.g. long-distance running.

Increased amounts of protein or raised doses of radiation or toxic substances in the kidneys may eventually lead to kidney damage.

International patent application No. WO91/04755, filed September 24, 1990, teaches methods to reduce non-target kidney retention of immunoconjugates, metabolites thereof, including other substances, such as peptides, by coadministration of a non-target moiety, such as lysine, or other amino acids having a free amino group. The application discloses only experimental results with mice showing that higher doses of lysine as a non-target reduction moiety improved results, i.e. the percentage of the injected immunoconjugate dose detected in the kidney is decreased. However, no information is provided concerning the possible toxicity of the method, and no data in humans were supplied. It is a known fact that there are differences in the physiology, including the renal physiology between mice and humans.

Additionally, it is known that infusion of amino acid solutions can induce side effects, including hyperkalemia (e.g. Ponce SP, et al. Medicine 64: 357-370, 1985, and Sartori S, et al. Recenti Progressi in Medicina 82(6): 275-277, 1991). This has been reported in particular for arginine (e.g. Massara F, et al. Diabete et Metabolisme 5(4):297-300, 1979), but it is also known in general medical practice for lysine, among others (Speck SP. in: Rakel RE, ed. Saunders Manual of Medical Practice; W.B. Saunders Company, Philadelphia, 2000, pp 885-888).

It is furthermore a known practice to use in patients an infusion of commercially available cocktails of various amino acids to lower the uptake of radiolabeled pharmaceuticals such as [¹¹¹In-DTPA-D-Phe¹]octreotide in the kidney (Hammond et al. in Br. J. Cancer 67, 1437-1439 (1993), Barone R, et al., J. Nucl. Med, 41: 94P (2000)). Such cocktails usually comprise a total amount of about 100 grams or more of various amino acids. In order to keep the osmolarity of such mixed amino acid solutions tolerable for human infusion, the total volume will be in the range of 2 liters.

This type of treatment has the observed disadvantageous side-effect that patients may suffer from severe vomiting, presumably caused by toxicity of the high total amount of amino acids administered. Additionally, the infusion of any volume of 2 liters within 4 hours, especially when it is hyperosmolar, can be dangerous to patients with impaired cardiac or renal function.

For the purpose of the invention, i.e. inhibiting renal uptake, it is necessary to reach a particular serum level of amino acids within a relatively short time, because excretion of the bulk of radiopharmaceuticals occurs within the first 4 to 6 hours after administration. During this time the radiopharmaceuticals may be taken up by the kidneys. It is thus not possible to avoid the side-effect of vomiting by administering the amino acid cocktail over a longer period of time.

In this connection a publication by Behr et al. [J. Nucl Med. 37(5): 829-833 (1996)] may be mentioned, wherein the reduction of the renal uptake of radiolabelled monoclonal antibody fragments by amino acids infusion is described. Also in this publication a cocktail of amino acids is infused, in a total volume of 2 liters and during approx. 2 hours, wherein the basic amino acids lysine (as glutamate) and arginine are administered in total amounts of 2.25 g and 5.00 g, respectively. According to the experience of the present inventors, such small dosages per treatment have appeared to be insufficiently effective in inhibiting the renal uptake of radiolabelled proteinaceous compounds in patients. In this connection it should be pointed out, that the authors of said Behr et al. publication consider their results as "still preliminary".
Infusion of a dosage of lysine, sufficient according to the present inventors, viz. of at least 15 grams, by administering said Behr et al. cocktail would lead to a too prolonged infusion period, viz. of at least 12 hours.

The information from the publication by Behr et al. is not suggestive for the present invention, neither alone nor in combination with the above-cited Hammond et al. publication [Br. J. Cancer 67: 1437-1439 (1993)], wherein the administration of 2 liters Synthamin 14 cocktail, containing approx. 170 grams total amino acids of which 9.86 g lysine and 35.2 g arginine and having an osmolarity of 880 mosmol/L, is described. In fact, Behr et al's publication does not contribute to a solution to the tolerability problem, as observed by the present inventors, viz. the adverse side-effect of vomiting by the patients during the administration of such amino-acid cocktails as described by Hammond et al.

Furthermore, it has been described by De Jong et al. in J. Nucl. Med 37(8): 1388-1392 (1996) that uptake in the rat kidneys of the [¹¹¹In-DTPA-D-Phe¹]octreotide can be inhibited by 50% by intravenous administration of the amino acid L-lysine alone (400 mg/kg). In a further article by Bernard et al. in J. Nucl. Med. 38: 1929-1933 (1997) it was demonstrated that also D-lysine is capable of reducing the rat renal uptake of [¹¹¹In-DTPA-D-Phe¹]octreotide and ⁹⁰Y-DOTA,Tyr³⁻octreotide.

However, treatment with lysine has also various disadvantages. It was for example found in humans that L-lysine in an effective total dose of 75 g (see examples for details) may lead to severe hyperkalemia which may result in acute and life-threatening cardiotoxicity in 50% of the patients.

In WO96/29087 it is also suggested to reduce kidney uptake of antibody fragment conjugates by administration of lysine or poly-lysine. It is taught in said application that toxicity of lysine can be avoided by using D-lysine because it does not occur naturally in humans or animals and is believed to be metabolically inert, thereby reducing the risk of toxic side-effects associated with the use of L-lysine. The prescribed dosage of monomeric lysine in this application is 1-200 g. The examples do not give results of treatments with humans with lysine alone. The only experiments in humans are performed with the commercially available amino acid solution Periamin X^{™}. According to the invention it is now shown that even 75 g of lysine is too high a dose. This already indicates that WO96/29087 may mention, but does not really enable the use of lysine in humans.

From the prior art it thus follows that reduction of renal uptake of unwanted proteinaceous materials can be achieved by either the use of amino acid cocktails that are commonly used as parenteral food supplements or with lysine alone. Either method has however the disadvantage that it may lead to toxic effects in humans.

It is therefore the object of the invention to provide a new improved way of inhibiting the renal tubular uptake of various types of proteinaceous molecules, such as proteins, peptides and antibodies.

This is achieved by the invention by the use of the combination of:
- a first amino acid which is a lysine selected from D-lysine, L-lysine or poly-lysine, or a pharmaceutically acceptable salt or carboxylic acid derivative thereof, and
- a second amino acid which is selected from the group consisting of arginine and ornithine, or a pharmaceutically acceptable salt or carboxylic acid derivative thereof,
for the preparation of a composition for inhibiting renal uptake of substances, in particular proteins or peptides, that may be damaging to the kidneys, and that are used for therapeutical or diagnostic purposes,
wherein said first amino acid is used in an amount of 15-35 grams, preferably 20-30 grams, more preferably about 25 grams per treatment, and wherein said second amino acid is used in an amount of 15-35 grams, preferably 20-30 grams, more preferably about 25 grams per treatment. Lysine and arginine are the preferred amino acids.

It was found that 50% inhibition in renal uptake of [¹¹¹In-DTPA-D-Phe¹]octreotide in rats could be achieved by D-Lysine or L-lysine at 400 mg/kg. L-arginine alone gave a reduction of 20-30% at an equimolar dose. In human studies 15, 21 and 40% reduction of kidney uptake of [¹¹¹In-DTPA-D-Phe¹]octreotide was reached using a dose of 25, 50 and 75 g L-lysine, respectively. The doses of 25 and 50 g L-lysine were well tolerated without any toxicity noted, but the 75 g L-lysine dose was associated with severe hyperkalemia in 50% of the patients. Hyperkalemia may result in acute and life-threatening cardiotoxicity.

The combination of L-lysine and L-arginine, however, shows a synergistic effect and is more effective than both compounds alone at equimolar concentrations in reducing renal uptake of radiolabeled peptides. Using the combination, lower doses of the two amino acids can therefore effectively inhibit the (radio)pharmaceutical renal uptake and simultaneously prevent hyperkalemia and cardiotoxicity. The normal serum potassium values lie between 3.5 and 5.3 mmol/L. Values between 5.3 and 6.0 mmol/L are a grey area. Values above 6.0 mmol/L are not acceptable, and cardiotoxicity may occur.

An additional advantage of the use of the two compounds of the invention is that the total infusion volume can be kept to 1 liter, which is safer than 2 liter in patients with compromised renal or cardiac function. In the cited literature, e.g. in the above Hammond et al. publication, only 2 liter volumes are used.

The treatment of the invention was found to have a lower impact on the increase in potassium level in serum than a treatment with 75 g lysine in 1500 mL. Whereas in the latter case 3 out of 6 patients had a maximum potassium level above the critical value of 6.0 mmol/L, the treatment of the invention resulted in only one out of eleven patients having a maximum potassium value of 6.0 mmol/L.

It is assumed that a possible mechanism of action of the invention is based on a blockade of megalin, a type I membrane glycoprotein of 4630 amino acids, 600 kDa, and having a pI of 4.6 that mediates renal tubular reabsorption. Megalin's mechanism of action is based on endocytosis through coated pit vesicles. Megalin transports positively charged molecules and is found on the brush border of proximal tubular cells, thyroid follicular cells, parathyroid cells, in the mammary gland, placenta, yolk sac and on lung type II pneumocytes.

The inhibition of renal uptake of proteins and peptides can be effected during therapy and diagnosis to protect the kidneys from detrimental side-effects of the therapeutic or diagnostic materials. In addition, when tissues surrounding the kidneys are subjected to localization with radiolabeled proteins or peptides, renal uptake of these radioactive materials may interfere with the scintigraphic visualization due to radiation from the kidneys that obscures the radiation from these surrounding tissues. Inhibition of accumulation of radioactivity in the kidneys is then also desirable.

It was also found according to the invention that after treatment with the combination of L-lysine plus L-arginine during radiotherapy with [¹⁷⁷Lu-DOTA,Tyr³] octreotate none of the 11 patients treated suffered from vomiting. In contrast, with the treatment with a cocktail of several amino acids (see examples for the details) 42% of the patients vomited who received radiotherapy with ⁹⁰Y-DOTA,Tyr³-octreotide. In 45% of these patients vomiting was very severe, i.e. 5 to 30 times. In another experiment it was found that in only 8% of treatments with the L-lysine and L-arginine combination vomiting occurred, mainly observed in one single patient, and this were probably not related to this combination.

The invention is suitable for inhibiting the renal uptake of all sorts of proteinaceous materials, such as proteins, peptides and monoclonal antibodies or their fragments. Renal uptake is to be avoided in case the proteinaceous materials are inherently toxic for the kidneys, and when they are coupled to a toxin, a radionuclide, a cytostatic agent or other potentially detrimental product. Renal uptake of nephrotoxic antibiotics or cytostatic agents per se is also to be prevented. In some cases the uptake by the kidneys of other, non-detrimental proteins can also be a problem. Protein is metabolized by the liver and excreted by the kidneys into the urine. A high protein load causes damage to these organs. In addition, diseases related to amino acid metabolism could lead to a protein overload in the kidneys.

Specific examples of diagnosis and therapies in which the composition of the invention could be used to inhibit or prevent renal uptake of the diagnostic or therapeutic agent are the use of radiolabelled peptides, including but not limited to octreotide and other somatostatin analogs, labelled with ¹¹¹In, ⁹⁰Y, ¹⁷⁷Lu, ¹³¹I or any other suitable radionuclide. Further examples are the diagnostic and therapeutic application of monoclonal antibodies or their fragments, nephrotoxic drugs such as antibiotics and cytostatic drugs.

In all these and other situations, the inhibition of the uptake of proteins or pharmaceuticals by the kidneys according to the invention can be beneficial.

The invention further relates to therapeutical compositions for the inhibition of the renal uptake of substances, in particular proteins or peptides, that may be damaging to the kidneys and that are used for therapeutical or diagnostic purposes, which composition comprises one or more pharmaceutically acceptable excipients, carriers or diluents and a combination of
- a first amino acid which is a lysine selected from D-lysine, L-lysine or polylysine, or a pharmaceutically acceptable salt or carboxylic acid derivative thereof, and
- a second amino acid which is selected from the group consisting of arginine and ornithine, or a pharmaceutically acceptable salt or carboxylic acid derivative thereof,
wherein said first amino acid is present in an amount of 15-35 grams, preferably 20-30 grams, more preferably about 25 grams per treatment, and wherein said second amino acid is present in an amount of 15-35 grams, preferably 20-30 grams, more preferably about 25 grams per treatment.

The above amounts are preferred for administration in 1 L over 4 hours. The total amount of the two compounds does not exceed 70 grams and is more preferably not more than 50 grams. The amounts given are intended for adults. For children the usual modifications can be made.

The preferred combination is such that the first compound is lysine, which may be either D-lysine, L-lysine or poly-lysine. The second compound is preferably arginine, which may be either D-arginine, L-arginine or poly-arginine.

The therapeutical composition of the present invention can be in an oral or parenteral dosage form. Parenteral dosage forms include intravenous, intraarterial, intraperitoneal, intramuscular and subcutaneous dosage forms, preferably intravenous dosage forms. Administration may be via a single or via multiple boluses, or by continuous or discontinuous infusion and is preferably by continuous infusion over 4 hours, starting about 30 minutes prior to administration of the radiopharmaceutical.

The combination of compounds of the present invention may be administered in any pharmaceutically acceptable solution. A solution is said to be pharmaceutically acceptable if its administration can be tolerated by a recipient patient. Sterile phosphate-buffered saline is one example of a pharmaceutically acceptable carrier, as is Ringer lactate, Hartman's solution or a mixture of glucose and saline. Other suitable carriers are well-known to those skilled in the art.

The present invention will be further illustrated in the examples that follow and that are not intended to limit the scope of the invention.

In the examples reference is made to the following figures:

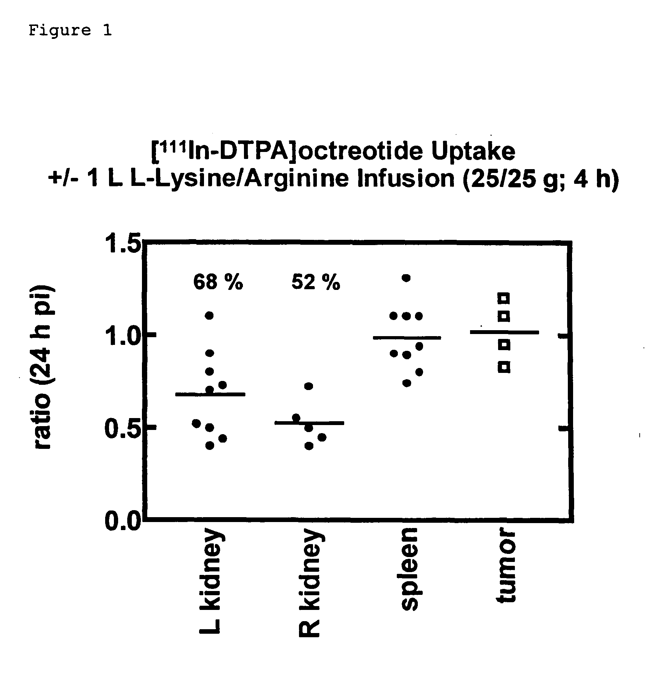

**Figure 1** shows the ratio between [¹¹¹In-DTPA-D-Phe¹]octreotide uptake in various organs in the presence or absence of 1 L lysine/arginine infusion (25/25 g; administered over 4 hours). Filled dots represent non-targeted accumulation of the radiopharmaceutical. Open squares represent the targeted accumulation of the radiopharmaceutical.

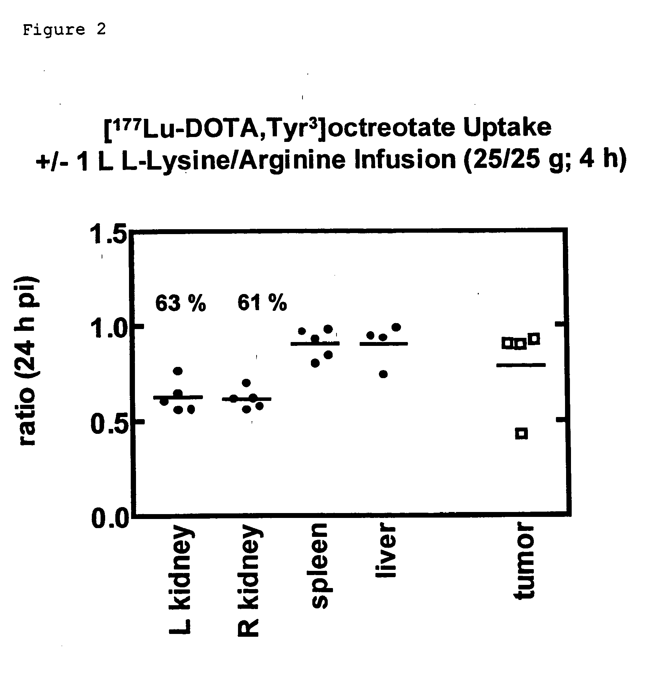

**Figure 2** shows the ratio between [^{177Lu}-DOTA, Tyr³]octreotate uptake in various organs in the presence or absence of 1 L lysine/arginine infusion (25/25 g; administered over 4 hours). Filled dots represent non-targeted accumulation of the radiopharmaceutical. Open squares represent the targeted accumulation of the radiopharmaceutical.

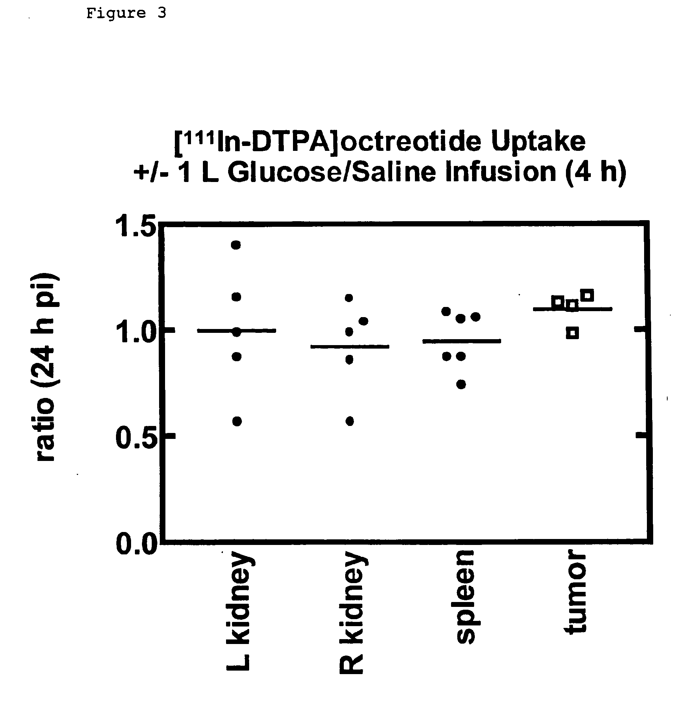

**Figure 3** shows the ratio between [¹¹¹In-DTPA-D-Phe¹]octreotide uptake in various organs in the presence or absence of 1 L glucose/saline solution, administered over 4 hours. Filled dots represent non-targeted accumulation of the radiopharmaceutical. Open squares represent the targeted accumulation of the radiopharmaceutical.

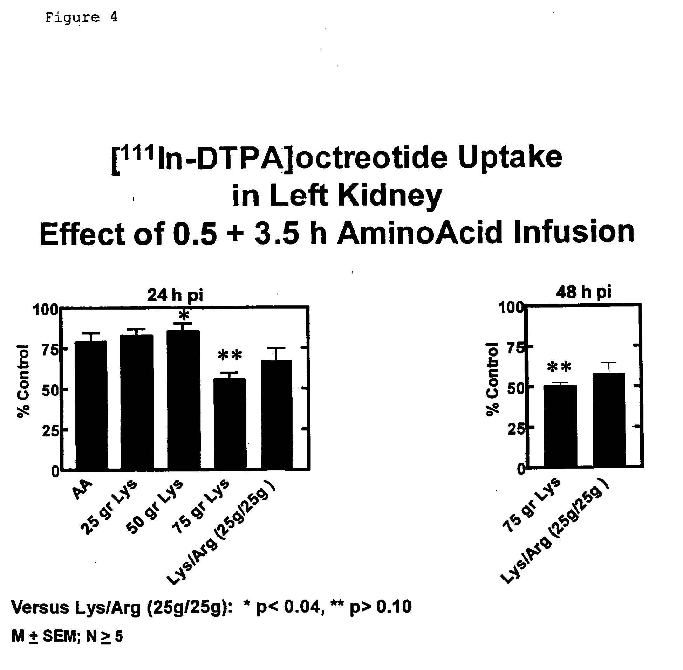

**Figure 4** shows between [¹¹¹In-DTPA-D-Phe¹]octreotide uptake in the left kidney after a 0.5 + 3.5 hour infusion of various amino acid compositions.

### EXAMPLES

### MATERIALS

### Composition of infusion fluids

### A. Lys/Arg

### Combination of L-lysine and L-arginine (total 1000 mL)

- L-arginine HCl 10% 250 mL (=25 grams of L-arginine)
- L-lysine HCl 5% 500 mL (=25 grams of L-lysine)
- HCl 25% is added to achieve pH 7.4
- NaCl 0.9% is added to achieve a total volume of 1000 mL
The osmolarity of this combination is ca. 400 mosmol/L
The total quantity of amino acids is 50 grams.

### B. Cocktail of various amino acids (total 2030 mL)

- Aminosteril N-Hepa 8% 1500 mL
- Ringer lactate 500 mL
- Magnesium sulfate (7-water) 10% 30 mL
The osmolarity of this combination is ca. 700 mosmol/L
The total quantity of amino acids is 124.5 grams.

Aminosteril N-Hepa 8% is a commercially available solution containing a mixture of amino acids (Fresenius AG, Gluckensteinweg 5, D-6380 Bad Homburg v.d. H., Germany). The composition is given herein below. Ringer lactate is a mineral solution containing NaCl 0.6%, CaCl₂.2H₂O 0.04%, Na-lactate 0.322% (Baxter B.V., Energielaan 3, NL-5405 AD Uden, The Netherlands)

**Composition of Aminosteril N Hepa 8% infusion fluid**

| | | | **Total in 1500 mL** | |
|---|---|---|---|---|
| **SUBSTANCE** | **QUANTITY (g/L)** | | | |
| L-isoleucine | 10,40 | | 15,600 | |
| L-leucine | 13,09 | | 19,635 | |
| L-lysinemonoacetate | 9,71 | (6.88) | 14,565 | (10.32) |
| (= 6,88 g L-lysine) | | | | |
| L-methionine | 1,10 | | 1,650 | |
| Acetylcysteine | 0,70 | (0.52) | 1,050 | (0.78) |
| (= 0,52 g L-cysteine) | | | | |
| L-fenylanaline | 0,88 | | 1,320 | |
| L-treonine | 4,40 | | 6,600 | |
| L-tryptophane | 0,70 | | 1,050 | |
| L-valine | 10,08 | | 15,120 | |
| L-arginine | 10,72 | | 16,080 | |
| L-histidine | 2,80 | | 4,200 | |
| Aminoacetic acid | 5,82 | | 8,730 | |
| (glycine) | | | | |
| L-alanine | 4,64 | | 6,960 | |
| L-proline | 5,73 | | 8,595 | |
| L-serine | 2,24 | | 3,360 | |
| Acetic acid | 4,42 | | | |
| **Total amino acids** | **83,01 g/L** | | **124,52 grams** | |
| Total nitrogen | 12,90 g/L | | | |
| **Osmolarity** | **770 mosm/L** | | **770 mosm/L** | |

### C.L-Lysine (total 500 mL) per unit

- L-lysine HCl 5% 500 mL (=25 grams of L-lysine)
- HCl 25% is added to achieve pH 7.4
The osmolarity of this solution is ca. 400 mosmol/L

For 50 grams of L-lysine 2 units (total 1000 mL) are used; for 75 grams of L-lysine 3 units (1500 mL).
Saline (NaCl 0.9%) or a mixture of glucose and saline (NaCl 0.45% + glucose 2.5%), can be added to increase the infusion volume.

All these infusion fluids are given over a period of 4 hours, with a maximum of 2.03 L per 4 hours. The rate of infusion can be constant over 4 hours, but initial high rates of infusion followed by lower rates of infusion are employed also. These infusion fluids can be given for periods up to 12 hours, with maximal average infusion rates of 2.03 L per 4 hours.

### EXAMPLE 1

### Effect of intravenous Lysine/Arqinine infusion on tissue uptake

A 1000 mL infusion containing 25 g L-lysine and 25 g L-arginine (**Lys/Arg;** see MATERIALS, paragraph A. for exact formulation) was administered intravenously to patients during 4 hours, starting 30 minutes prior to administration of the radioligand. One radioligand was [¹¹¹In-DTPA-D-Phe¹]octreotide (¹¹¹In-pentetreotide, OctreoScan®, Mallinckrodt Medical, Petten, The Netherlands) in a diagnostic amount of 220 MBq (9 patients). Each patient was also investigated on a separate occasion with 220 MBq ¹¹¹In-pentetreotide, but without any infusion. The other radioligand was [¹⁷⁷Lu-DOTA,Tyr³]octreotate in a therapeutic amount of 1850 MBq (5 patients). Each patient was also investigated on a separate occasion with 1850 MBq [¹⁷⁷Lu-DOTA,Tyr³]-octreotate, but without any infusion. The control experiment (5 patients) consisted of infusion of 1000 mL neutral infusion fluid without the amino acids (NaCl 0.45% plus Glucose 2.5%)over 4 hours, starting 30 min. prior to the injection of 220 MBq ¹¹¹In-pentetreotide.

24 Hours post infusion the uptake of the radioligand in the kidneys, spleen and liver was measured using planar scintigraphy and dosimetry, and for each patient the ratio between uptake in these organs during infusion with **Lys/Arg** and without **Lys/Arg** was calculated (Figures 1, 2 and 3).

In the 9 patients who received a diagnostic dose of 220 MBq of ¹¹¹In-pentetreotide, during **Lys/Arg** infusion the mean uptake of the radioligand in the kidneys was reduced to 68% (left) and 52% (right) of their own control without **Lys/Arg** (Figure 1).

In the 5 patients who received a therapeutic dose of 1850 MBq [¹⁷⁷Lu-DOTA,Tyr³]octreotate, during **Lys/Arg** infusion the mean uptake of the radioligand in the kidneys was reduced to 63% (left) and 61% (right) of their own control without **Lys/Arg** (Figure 2).

The control experiment showed no significant differences in renal uptake of ¹¹¹In-pentetreotide whether 1000 mL of (neutral) infusion fluid was administered or not, thus ruling out the infusion volume itself as a contributing factor to reduce renal uptake of ¹¹¹Inpentetreotide (Figure 3).

From these combined results it follows that there is a 40% decrease of ¹¹¹In-pentetreotide or [¹⁷⁷Lu-DOTA,Tyr³]octreotate uptake in the kidneys during **Lys/Arg** infusion as compared to the control.

### EXAMPLE 2

### Effect of Lys/Arg infusion on serum potassium levels

In an experiment of the invention 11 patients received an infusion over 4 hours of a solution containing 25 g L-lysine and 25 g L-arginine in 1000 mL (**Lys/Arg;** see MATERIALS, paragraph A. for exact formulation). The potassium level of these patients was measured in serum at t=0 hours and after 0.5, 1, 2, 4 and 5 hours. The results are shown in table 1 and figure 4.

**Table 1**

| Potassium level in serum during infusion of 1000 mL Lys/Arg over 4 hours, starting 30 min. prior to the administration of ¹¹¹In-pentetreotide in a diagnostic amount of 220 MBq. | | | | | | | |
|---|---|---|---|---|---|---|---|
| patient no. | 0 | 0.5 | 1 | 2 | 4 | 5 | max. differen ce |
| 1 | 4.6 | 4.6 | 4.9 | 5.4 | 5.5 | 5.4 | 0.9 |
| 2 | 4.2 | 4.1 | 4.6 | | 4.8 | | 0.7 |
| 3 | 4.3 | 4.5 | 4.7 | 4.9 | 5.4 | 4.8 | 1.1 |
| 4 | 4.5 | 4.7 | 5.3 | 6.0 | 5.7 | | 1.5 |
| 5 | 4.5 | 4.5 | | 5.0 | 5.3 | | 0.8 |
| 6 | 4.1 | 4.2 | | 5.1 | 5.2 | 5.3 | 1.1 |
| 7 | 4.4 | 4.1 | | 4.5 | 5.0 | | 0.6 |
| 8 | 3.7 | 3.3 | | 3.9 | 4.5 | 4.4 | 0.8 |
| 9 | 3.0 | 3.0 | | 3.6 | 4.0 | | 1 |
| 10 | 4.3 | 4.3 | | 4.8 | 5.5 | | 1.2 |
| 11 | 4.5 | n.d. | 4.4 | 4.9 | 4.8 | | 0.4 |
| Average | 4.2 | 4.1 | 4.8 | 4.8 | 5.1 | | 0.9 |

As a control 6 patients received 75 g L-lysine in 1500 mL during 4 hours (see MATERIALS, paragraph C. for exact formulation). Their maximum serum potassium levels are given in Table 2.

**Table 2**

| Maximum,potassium level in serum during infusion of 75 g L-lysine in 1500 mL over 4 hours, starting 30 min. prior to the administration of ¹¹¹In-pentetreotide in a diagnostic amount of 220 MBq (patients 12, 13, 14, 15), or in a therapeutic amount of 7 GBq (patients 16, 17). | |
|---|---|
| patient | maximum potassium (K) level |
| 12 | 5.2 |
| 13 | **6.8** |
| 14 | **6.7** |
| 15 | 5.4 |
| 16 | 5.0 |
| 17 | **6.3** |

Patient no. 17 showed transient muscle weakness, the other patients had no symptoms related to the infusion.

### EXAMPLE 3

### Effect of different amino acid preparations on [¹¹¹In-DTPA]octreotide uptake in the kidney

At least 5 patients received one of the following amino acid preparations:
1. AA (2030 mL cocktail of various amino acids; see MATERIALS, paragraph B, for the source and details)
2. 25 g L-lysine in 500 mL (see MATERIALS, paragraph C, for exact formulation)
3. 50 g lysine in 2000 mL (see MATERIALS, paragraph C, for exact formulation)
4. 75 g lysine in 1500 mL (see MATERIALS, paragraph C, for exact formulation)
5. 25 g L-lysine and 25 g L-arginine in 1000 mL (invention), (**Lys/Arg;** see MATERIALS, paragraph A. for exact formulation).

The preparations were given intravenously over 4 hours starting 30 minutes before treatment with the radioligand started. The radioligand was ¹¹¹Inpentetreotide, either in a diagnostic amount of 220 MBq, or in a therapeutic amount, 7 - 11 GBq. Each patient was his or her own control. The control consisted of treatment with the radioligand but without an amino acid preparation.

At 24 and 48 hours post infusion the ratio between tissue uptake in the left kidney in patients receiving an amino acid composition and the controls was determined by planar scintigraphy and dosimetry.

Figure 4 gives the results of this study, which also includes the data obtained in patients described in examples 1 and 2. Although 75 g L-lysine leads to the highest uptake reduction, it was found that it resulted in severe hyperkalemia (Table 2). No hyperkalemia was found in the second best amino acid composition comprising 25 g L-lysine plus 25 g L-arginine (Table 1). Moreover, it was found that more patients vomited that received the AA compositions than patients receiving L-lysine or Lys/Arg(see also EXAMPLES 4, 5, 6).

### EXAMPLE 4

### Effect of prior art amino acid composition on vomiting

26 patients received 1 to 5 therapeutic doses of 1-10 GBq ⁹⁰Y-DOTA,Tyr³-octreotide, with concomitant infusion of a 2030 mL cocktail of various amino acids in 4 hours, starting 30 min. before the administration of the radiopharmaceutical (see MATERIALS, paragraph B. for exact details). In 11 of these 26 patients (42%), [being 20 of 84 treatments (24%)] one or more episodes of vomiting occurred, up to 30 episodes of severe vomiting per treatment.

In one of these 11 patients the vomiting was so severe during the 2 treatments with the above described regimen, that it was decided to use an infusion with 50 g of L-lysine instead of the infusion with the cocktail of various amino acids concomitant with his 3rd and 4th therapy with ⁹⁰Y-DOTA,Tyr³-octreotide. Only one episode of vomiting occurred after his 3rd treatment and no vomiting at all after his 4th treatment.

### EXAMPLE 5

### Effect of lysine on vomiting

8 patients received multiple therapeutic doses of 7-11 GBq ¹¹¹In-pentetreotide. All received once a concomitant infusion of a 2030 mL cocktail of various amino acids in 4 hours, starting 30 min. before the administration of the radiopharmaceutical (see MATERIALS, paragraph B. for exact details). In 4 of these 8 patients (50%) vomiting occurred.

In these same 8 patients only 3 episodes (9%) of vomiting occurred during 22 treatments with 7-11 GBq ¹¹¹In-pentetreotide while they received 25 or 50 grams of L-lysine in 500 to 2000 mL (see MATERIALS, paragraph C. for exact details) over 4 hours, starting 30 min. before administration of the radiopharmaceutical, as concomitant infusions.

In these same 8 patients only 3 episodes (6%) of vomiting occurred during 52 treatments while they received no concomitant infusion at all.

### EXAMPLE 6

### Effect of Lys/Arg on vomiting

In an experiment of the invention 11 patients received an infusion over 4 hours of a solution containing 25 g L-lysine and 25 g L-arginine in 1000 mL (**Lys/Arg;** see MATERIALS, paragraph A. for exact formulation). Thirty min. after the start of the infusion they received a diagnostic dose of 220 MBq ¹¹¹Inpentetreotide. Ten patients had no symptoms during the infusion, in particular no vomiting. One patient had subfebrile temperature, malaise and nausea already before the start of the infusion, presumably caused by tumor necrosis. During the **Lys/Arg** infusion she vomited twice. This patient has tolerated the **Lys/Arg** infusion without any problems on all 4 later occasions, while receiving a therapeutic dose of 10-11 GBq ¹¹¹In-pentetreotide.

Five patients received the **Lys/Arg** infusion in the same manner as described above, but they received a therapeutic dose of 1850 MBq [¹⁷⁷Lu-DOTA,Tyr³]octreotate, 30 min. after the start of the **Lys/Arg** infusion. None of these 5 patients had any symptoms at all, in particular no vomiting.

Until now the **Lys/Arg** infusion, 1000 mL in 4 hours concomitant with therapeutic doses of radioactive labelled peptides, has been administered to more than 30 patients, in total more than 80 occasions. Vomiting occurs in less than 5% of the occasions, and if it occurs it is most probably due to other causes.

From these studies described in EXAMPLES 4, 5 and 6 it was concluded that the infusion of 2030 mL containing a cocktail of various amino acids causes frequent vomiting, which is unacceptable for routine clinical circumstances. On the other hand, vomiting is seldomly observed either with L-lysine infusions or with **Lys/Arg** infusion.

## Claims

1. Use of the combination of:
- a first amino acid which is a lysine selected from D-lysine, L-lysine or poly-lysine, or a pharmaceutically acceptable salt or carboxylic acid derivative thereof, and
- a second amino acid which is selected from the group consisting of arginine and ornithine, or a pharmaceutically acceptable salt or carboxylic acid derivative thereof,
for the preparation of a composition for inhibiting renal uptake of substances, in particular proteins or peptides, that may be damaging to the kidneys, and that are used for therapeutical or diagnostic purposes,
wherein said first amino acid is used in an amount 15-35 grams, preferably 20-30 grams, more preferably about 25 grams per treatment, and wherein said second amino acid is used in an amount of 15-35 grams, preferably 20-30 grams, more preferably about 25 grams per treatment.

2. Use as claimed in claim 1, wherein the first amino acid is lysine and the second amino acid is arginine.

3. Use as claimed in claim 1 or 2, wherein both amino acids are used in amounts of about 25 grams per treatment.

4. Therapeutical composition for the inhibition of the renal uptake of substances, in particular proteins or peptides, that may be damaging to the kidneys and that are used for therapeutical or diagnostic purposes, which composition comprises one or more pharmaceutically acceptable excipients, carriers or diluents and a combination of
- a first amino acid which is a lysine selected from D-lysine, L-lysine or poly-lysine, or a pharmaceutically acceptable salt or carboxylic acid derivative thereof, and
- a second amino acid which is selected from the group consisting of arginine and ornithine, or a pharmaceutically acceptable salt or carboxylic acid derivative thereof,
wherein said first amino acid is present in an amount of 15-35 grams, preferably 20-30 grams, more preferably about 25 grams per treatment, and wherein said second amino acid is present in an amount of 15-35 grams, preferably 20-30 grams, more preferably about 25 grams per treatment.

5. Therapeutical composition as claimed in claim 4, wherein the first amino acid is lysine and the second amino acid is arginine.

6. Therapeutical composition as claimed in claim 4 or 5, wherein both amino acids are present in amounts of about 25 grams per treatment.

7. Therapeutical composition as claimed in claims 4-6, wherein the two amino acids or their respective derivatives are present in about 1 L infusion fluid.

8. Use of a combination of a first amino acid and a second amino acid or their respective derivatives, as defined in claim 1, for the manufacture of a therapeutical composition as claimed in claim 4, for inhibiting the renal uptake of proteins or peptides, that are used for therapeutical or diagnostic purposes, in a subject.

## Patentansprüche

1. Verwendung der Kombination aus:
- einer ersten Aminosäure, die ein Lysin ist, ausgewählt aus D-Lysin, L-Lysin oder Polylysin oder ein pharmazeutisch akzeptables Salz oder Carbonsäurederivat davon, und
- einer zweiten Aminosäure, ausgewählt aus der Gruppe aus Arginin und Omithin oder ein pharmazeutisch akzeptables Salz oder Carbonsäurederivat davon, zur Herstellung einer Zusammensetzung zur Inhibierung der renalen Aufnahme von Substanzen, insbesondere von Proteinen oder Peptiden, die für die Nieren schädigend sein können, und die für therapeutische oder diagnostische Zwecke verwendet werden,
wobei die erste Aminosäure in einer Menge von 15 bis 35 g, vorzugsweise 20 bis 30 g, am meisten bevorzugt von ungefähr 25 g pro Behandlung verwendet wird, und wobei die zweite Aminosäure in einer Menge von 15 bis 35 g, vorzugsweise 20 bis 30 g, am meisten bevorzugt von ungefähr 25 g pro Behandlung verwendet wird.

2. Verwendung wie in Anspruch 1 beansprucht, wobei die erste Aminosäure Lysin ist und die zweite Aminosäure Arginin ist.

3. Verwendung wie in Anspruch 1 oder 2 beansprucht, wobei beide Aminosäuren in Mengen von ungefähr 25 g pro Behandlung verwendet werden.

4. Therapeutische Zusammensetzung zur Inhibierung der renalen Aufnahme von Substanzen, insbesondere von Proteinen oder Peptiden, die für die Nieren schädigend sein können, und die für therapeutische oder diagnostische Zwecke verwendet werden, wobei die Zusammensetzung einen oder mehrere pharmazeutisch akzeptable Hilfsstoffe, Träger oder Verdünnungsmittel und eine Kombination von
- einer ersten Aminosäure, die ein Lysin ist, das ausgewählt ist aus D-Lysin, L-Lysin oder Polylysin oder ein pharmazeutisch akzeptables Salz oder Carbonsäurederivat davon, und
- einer zweiten Aminosäure, die ausgewählt ist aus der Gruppe aus Arginin und Ornithin oder ein pharmazeutisch akzeptables Salz oder Carbonsäurederivat davon,
wobei die erste Aminosäure in einer Menge von 15 bis 35 g, vorzugsweise 20 bis 30 g, am meisten bevorzugt von ungefähr 25 g pro Behandlung vorhanden ist und wobei die zweite Aminosäure in einer Menge von 15 bis 35 g, bevorzugt 20 bis 30 g, am meisten bevorzugt von ungefähr 25 g pro Behandlung vorhanden ist.

5. Therapeutische Zusammensetzung wie in Anspruch 4 beansprucht, wobei die erste Aminosäure Lysin ist und die zweite Aminosäure Arginin ist.

6. Therapeutische Zusammensetzung wie in Anspruch 4 oder 5 beansprucht, wobei beide Aminosäuren in Mengen von ungefähr 25 g pro Behandlung vorhanden sind.

7. Therapeutische Zusammensetzung wie in Ansprüchen 4-6 beansprucht, wobei die beiden Aminosäuren oder ihre entsprechenden Derivate in ungefähr 1 1 Infusionsflüssigkeit vorhanden sind.

8. Verwendung einer Kombination einer ersten Aminosäure und einer zweiten Aminosäure oder deren entsprechenden Derivate, wie in Anspruch 1 definiert, zur Herstellung einer therapeutischen Zusammensetzung wie in Anspruch 4 beansprucht, zur Inhibierung der renalen Aufnahme von Proteinen oder Peptiden, die für therapeutische oder diagnostische Zwecke in einem Subjekt verwendet werden.

## Revendications

1. Application de la combinaison de :
- un premier aminoacide qui est une lysine choisie parmi la D-lysine, la L-lysine et la poly-lysine ou un sel ou dérivé d'acide carboxylique de celle-ci acceptable pharmaceutiquement, et
- un second aminoacide qui est choisi dans le groupe constitué par l'arginine et l'ornithine ou un sel ou dérivé d'acide carboxylique de celui-ci acceptable pharmaceutiquement,
à la préparation d'une composition destinée à inhiber le prélèvement rénal de substance, en particulier de protéines ou de peptides, qui peuvent être nuisibles pour les reins, et qui sont utilisés dans des buts thérapeutiques ou de diagnostic,
dans laquelle le premier aminoacide est utilisé en quantité comprise entre 15 et 35 g et de préférence entre 20 et 30 g et plus avantageusement d'environ 25 g par traitement, et dans laquelle le second aminoacide est utilisé en quantité comprise entre 15 et 35 g, de préférence entre 20 et 30 g et plus avantageusement d'environ 25 g par traitement.

2. Application selon la revendication 1, dans laquelle le premier aminoacide est la lysine et le second aminoacide est l'arginine.

3. Application selon la revendication 1 ou 2, dans laquelle les deux aminoacides sont utilisés en quantité d'environ 25 g par traitement.

4. Composition thérapeutique destinée à inhiber le prélèvement rénal de substances, en particulier de protéines ou de peptides, qui peuvent être nuisibles pour les reins et qui sont utilisés dans des buts thérapeutiques ou de diagnostic, la composition comprenant un ou plusieurs excipients, véhicules ou diluants pharmaceutiquement acceptables, et une combinaison de :
- un premier aminoacide qui est une lysine choisie parmi la D-lysine, la L-lysine et la poly-lysine ou un sel ou dérivé d'acide carboxylique de celle-ci acceptable pharmaceutiquement, et
- un second aminoacide qui est choisi dans le groupe constitué par l'arginine et l'ornithine ou un sel ou dérivé d'acide carboxylique de celui-ci acceptable pharmaceutiquement,
dans laquelle le premier aminoacide est utilisé en quantité comprise entre 15 et 35 g et de préférence entre 20 et 30 g et plus avantageusement d'environ 25 g par traitement, et dans laquelle le second aminoacide est utilisé en quantité comprise entre 15 et 35 g, de préférence entre 20 et 30 g et plus avantageusement d'environ 25 g par traitement.

5. Composition thérapeutique selon la revendication 4, dans laquelle le premier aminoacide est la lysine et le second aminoacide est l'arginine.

6. Composition thérapeutique selon la revendication 4 ou 5, dans laquelle les deux aminoacides sont présents en quantité d'environ 25 g par traitement.

7. Composition thérapeutique selon l'une quelconque des revendications 4 à 6, dans laquelle les deux aminoacides ou leurs dérivés respectifs sont présents dans un fluide d'infusion d'environ 1 l.

8. Application d'une combinaison d'un premier aminoacide et d'un second aminoacide ou de leurs dérivés respectifs tels que définis dans la revendication 1, à la fabrication d'une composition thérapeutique selon la revendication 4, pour l'inhibition du prélèvement rénal de protéines ou de peptides qui sont utilisés dans des buts thérapeutiques ou de diagnostic dans un sujet.
